# EUROPÄISCHE PATENTSCHRIFT

(11) **EP 0 427 035 B1**
(45) Veröffentlichungstag und Bekanntmachung des Hinweises auf die Patenterteilung: **10.05.1995**
(21) Anmeldenummer: 90120176.4
(22) Anmeldetag: 20.10.1990
(51) Int. Cl.: C12N 15/67, C12N 15/53, C12N 15/73, C12N 1/00, C12N 9/04, C12P 1/00

(54) **Überexpression von Proteinen in rekombinanten Wirtszellen**
Protein overexpression in recombinant host cells
Surexpression de protéines dans des cellules hôtes recombinantes

(30) Priorität: 02.11.1989 DE 3936408
(43) Veröffentlichungstag der Anmeldung: 15.05.1991
(73) Patentinhaber: MERCK PATENT GmbH, D-64271 Darmstadt (DE)
(72) Erfinder: Heinrich, Martin, Dr., W-6100 Darmstadt (DE); Ebeling, Wolfgang, W-6101 Bickenbach (DE); Brümmer, Wolfgang, Dr., W-6146 Alsbach-Hähnlein 1 (DE)

(56) Entgegenhaltungen:
- EP-A- 0 285 949
- EP-A- 0 290 768
- WO-A-85/03522
- JOURNAL OF BIOTECHNOLOGY, Band 5, Nr. 4, Mai 1987, Seiten 237-253, Amsterdam, NL; S. SUGIMOTO et al.: "Hyperproduction of phenylalanine by Escherichia coli: application of temperature-controllable expression vector carrying the represso-promoter system of bacteriophage lambda", S. 246, Absatz 2, S. 247, Tabel 1, Absatz 1, S. 248-252
- NUCLEIC ACIDS RESEARCH, Band 11, Nr. 14, 1983, Seiten 4677-4688, IRL Press, Oxford, GB; E. REMAUT et al.: "Inducible high level synthesis of mature human fibroblast interferon in Escherichia coli"

## Beschreibung

Die Erfindung betrifft ein Verfahren zur Erzielung hoher, über einen längeren Zeitraum andauernder, temperaturinduzierter Expressionsraten von Proteinen/Enzymen in rekombinanten Wirtszellen, insbesondere E. coli, mittels aufeinander abgestimmter Kultivierungsparameter wie Zelldichte, Induktionstemperatur und zugesetzter aminosäurehaltiger Substrate.

Ziel eines jeden gentechnologischen Verfahrens ist es, vor allem möglichst hohe Ausbeuten an rekombinantem Produkt zu erhalten. Hierfür hat sich bislang ein zweiphasiges Verfahren bewährt. Dazu strebt man zunächst ein optimales Wachstum der Zellen an, um eine hohe Biomasse und Plasmidmenge zu erhalten. Gleichzeitig muß darauf geachtet werden, daß bei gutem Wachstum nicht zuviel Acetat angehäuft wird. In dieser ersten Phase ist eine nennenswerte Produktion des rekombinanten Proteins unerwünscht, da das Zellenwachstum durch übermäßigen Substratverbrauch und/oder durch die Überproduktion des zuweilen zusätzlich toxisch wirkenden rekombinanten Proteins teilweise stark reduziert wird. Aus diesem Grund ist es wichtig, daß ein regulierbares Expressionssystem zur Verfügung steht. In einem solchen System ist der Promotor während der Wachstumsphase der Zellen reprimiert, so daß es zu keiner Produktion von rekombinantem Protein kommt. In einer zweiten Phase bei einer hohen Zelldichte bzw. Biomasse wird der Promotor dereprimiert und die Expression dadurch induziert. Es sind zahlreiche regulierbare Expressionssysteme für Mikroorganismen bekannt, bei denen die Induktion der Proteinexpression durch Temperaturshift oder chemische Induktion bewerkstelligt werden kann. Einer der bekanntesten und effektivsten Promotoren für E. coli ist der Lambda-P_{L}-promotor, der beispielsweise in der EP-OS-00 41 767 beschrieben ist. Durch Temperaturshift auf etwa 42 °C wird der den Promotor blockierende C1-Repressor thermisch inaktiviert und die Transkription des Strukturgens somit freigegeben (Phase 2). Viele Enzyme und andere Proteine sind mit einer solchen zweiphasigen Standardprozedur in E. coli schon exprimiert worden, so z. B. EcoRI-Restriktionsendonuklease (Bottermann et al., Biotechnol. Bioeng. 27 (1985), S. 1320), α-Amylase (Reinikainen et al., Biotechnol. Lett. 10 (3) (1988), S. 149), Mutarotase (EP-OS-03 07 730) oder Glucosedehydrogenase (EP-OS-02 90 768). Wachstum und Expression müssen dabei nicht notwendigerweise in einem Reaktor zeitlich hintereinander geschaltet werden, sondern können auch räumlich getrennt in zwei hintereinander geschalteten Reaktoren ablaufen.

Die geschilderte, bislang praktizierte Verfahrenstechnik, die auch in abgewandelter Form und mit anderen regulierbaren Promotorsystemen für andere Bakterienarten wie Bacillus angewendet wird, weist aber nicht selten Nachteile auf, die in speziellen Fällen das Verfahren unpraktikabel machen können. So wird mit steigender Anzahl von Plasmid-Replikationen und damit verbundener erhöhter Überexpression von Protein in den meisten Fällen eine teilweise starke Abnahme der Wachstumsrate der rekombinanten Wirtszellen beobachtet (z. B. Bentley und Kompala; Biotechn. Bioeng. 33 (1989) S. 49).

Weiterhin ist bekannt, daß eine Derepression des Promotors z. B. durch Temperaturshift zu einer starken Einschränkung des Wachstums und zu hohen Plasmidverlusten führen kann (z. B. Siegel und Ryu, Biotechn. Bioeng. 27 (1985), S. 28). Bemerkenswert ist zudem die häufig gemachte Beobachtung (z. B. Peretti und Bailey, Biotechn. Bioeng. 32 (1988), S. 418), daß es unmittelbar nach Promotorinduktion zu einer zunächst zwar sehr hohen aber meist nur kurzzeitigen, dann jedoch mehr oder weniger schnell abklingenden Biosyntheserate des rekombinanten Proteins kommt. All diese Effekte bewirken, daß die Nettosyntheserate limitiert ist durch die abnehmende Wachstumsrate nach erfolgter Promotorinduktion. So ist nicht nur die Ausbeute an rekombinantem Protein, absolut gesehen, oftmals unzureichend, sondern es müssen ständig neue rekombinante Wirtszellen kultiviert werden, da die alten Zellen nach relativ kurzer Zeit unproduktiv und meist nicht mehr regenerierbar sind. Dies ist ökonomisch von Nachteil.

Es bestand somit die Aufgabe bei der Produktion von Proteinen bzw. Enzymen mit Hilfe rekombinanter, regulierbare Promotoren enthaltender Wirtszellen durch Optimierung der Kultivierungsparameter die oben genannten Nachteile zu beseitigen und über einen großen Zeitraum hohe Biosyntheseraten des rekombinanten Proteins zu ermöglichen.

Es wurde nun gefunden, daß die Ausbeuten/Aktivitäten von rekombinant hergestellten Proteinen/Enzymen sich um das 5- bis 10fache steigern lassen und die Biosynthese über einen längeren Zeitraum als bisher auf einem hohen Niveau aufrecht erhalten werden kann, wenn die die Protein/Enzym-Expression einleitende Promotorinduktion (1) nicht erst, wie bislang praktiziert, bei einer maximalen sondern bereits bei einer mittleren Biomasse in einer relativ frühen Wachstumsphase der rekombinanten Wirtszellen, (2) bei einer Induktionstemperatur von unter 40 °C und (3) in Anwesenheit von zugesetzten aminosäurehaltigen Substraten durchgeführt wird. So lassen sich beispielsweise für das gentechnisch hergestellte Enzym Glucosedehydrogenase Aktivitäten von 200 bis 500, vorzugsweise von 250 bis 350 U/ml Kulturbrühe und einer spezifischen Aktivität von 100 bis 300, vorzugsweise von 150 bis 250 U/mg Protein erzielen.

Gegenstand der Erfindung ist somit ein Verfahren zur Herstellung von Proteinen durch Kultivierung rekombinanter Wirtszellen in einer Nährlösung und temperaturinduzierte Expression durch den Lambda-P_{L}-Promoter und nachfolgende Isolierung der Proteine, dadurch gekennzeichnet, daß die Expression der Proteine
a) bei einer Biomasse der rekombinanten Wirtszellen, die 5 bis 40 % der maximal erreichbaren Biomasse entspricht,
b) bei einer Induktions- bzw. Expressionstemperatur von unterhalb 40 °C, und
c) in Anwesenheit von unmittelbar vor oder nach zugesetzten Aminosäuren und/oder Hefeextrakten
durchgeführt wird.

Gegenstand der Erfindung ist insbesondere ein entsprechendes Verfahren, das dadurch gekennzeichnet ist, daß die rekombinanten Wirtszellen E. coli-Zellen sind, die Expression der Proteine durch den Lambda-P_{L}-Promotor kontrolliert wird, und das zugesetzte Gemisch aus Aminosäuren mindestens Phenyl alanin, Tyros in und Tryptophan enthält.

Gegenstand der Erfindung ist insbesondere auch ein entsprechendes Verfahren, das dadurch gekennzeichnet ist, daß das exprimierte Protein Glucosedehydrogenase ist.

Zur Durchführung des erfindungsgemäßen Verfahrens eignen sich prinzipiell alle rekombinanten transformierten Wirtszellen mikrobieller Herkunft, in denen das zur Expression zu bringende Strukturgen unter Kontrolle mindestens eines regulierbaren durch Temperatur induzierbaren Promotors steht. Als Mikroorganismen sind insbesondere E. coli und Bacillus-Arten geeignet. Besonders bevorzugt sind rekombinante E. coli-Wirtszellen. Als regulierbare Promotoren sind alle in dem betreffenden Mikroorganismus effektiven Promotoren geeignet, insbesondere der E. coli Lambda-P_{L}-Promotor, der entsprechende lac-, trp-oder tet-Promotor oder auch Hybridpromotoren sofern sie temperaturregulierbar sind. Besonders bevorzugt ist der aus der EP-OS-001767 bekannte Lambda-P_{L}-Promotor der durch Temperaturerhöhung auf 42 °C induziert werden kann.

Die Art und Herkunft des zur Expression zu bringenden Strukturgens spielt bei der Durchführung des erfindungsgemäßen Verfahrens keine entscheidende Rolle. Prinzipiell ist somit jedes zur Expression in dem betreffenden Mikroorganismus befähigtes Strukturgen geeignet.

Die vorteilhaften Eigenschaften des erfindungsgemäßen Verfahrens müssen immer im direkten Vergleich zum ansonsten identischen Verfahren des Standes der Technik gesehen werden, d. h. gegenüber der Expression des gleichen Strukturgens bei identischer Plasmidkonstruktion im gleichen Mikroorganismus. Beispiele für geeignete Strukturgene sind Glucosedehydrogenase-Gene, das Mutarotase-Gen und die Gene für Sorbitdehydrogenase, Chloramphenicolacetyltransferase und Dihydrofolatreduktase.

Das erfindungsgemäße Verfahren wird bevorzugt wie folgt durchgeführt.

Als rekombinanter Mikroorganismus dient z. B. ein beliebiger E. coli-Stamm (z. B. E. coli K 12 oder E. coli N 100), der mit einem entsprechenden Expressionsvektor transformiert wurde. Vorzugsweise steht das Strukturgen unter Kontrolle des temperaturinduzierbaren Lambda-P_{L}-Promotors.

In einer bevorzugten Ausführungsform wird der E. coli-Stamm N 100/pRK 248/pJH 115 (DSM 4047) eingesetzt. Der Stamm ist aus der EP-OS-02 90 768 bekannt und ist mit dem Vektor pJH 115 transformiert worden. Plasmid pJH 115 enthält das Glucosedehydrogenase-Gen aus Bacillus megaterium.

Der entsprechende E. coli-Stamm wird nach Standardverfahren in bekannten und hierfür üblichen Anzuchtmedien kultiviert. Das Wachstum der Zellen wird in bekannter Weise über Messung der optischen Dichte bzw. der Trübung des Kulturmediums spektralphotometrisch, vorzugsweise bei 578 nm, verfolgt. Erfindungsgemäß werden die Zellen nur bis zu einer mittleren Biomasse wachsen gelassen (frühe bis mittlere Wachstumsphase), ehe der Temperaturshift vollzogen wird. Sie entspricht 5 bis 40 %, vorzugsweise 10 bis 20 % der maximal erreichbaren Biomasse des betreffenden rekombinanten Wirtsstammes unter den gegebenen Kultivierungsbedingungen. In der Regel ist dies gleichzusetzen mit einer einer bei 578 nm gemessenen Trübung von 2,0 bis 5,5 OD, vorzugsweise von 2,5 bis 4,5 OD entsprechenden Biomasse. Im Normalfall können maximale Biomassen erreicht werden, die einer Trübung von 8 bis 10 OD (578 nm) entsprechen. In Ausnahmefällen können jedoch auch schon deutlich niedrigere OD-Werte für maximal erreichbare Zelldichten gemessen werden. Auch spielt die jeweils verwendete Meßapparatur (Küvettengröße, Spektralphotometer etc.) eine gewisse Rolle. Erfindungswesentlich ist aber stets, daß die Aktivierung des Promotors durch Temperaturerhöhung und die damit in Gang gesetzte Proteinexpression bei einer Biomasse begonnen wird, die, wie oben genannt, deutlich unter der maximal erreichbaren Zelldichte liegt.

Die Temperatur in der Wachstumsphase (keine Proteinexpression) wird zwischen 25 und 35 °C, vorzugsweise zwischen 28 und 32 °C gehalten. Bei der gewünschten Zelldichte wird die Temperatur auf 37 bis 40 °C, vorzugsweise auf 38 bis 39 °C erhöht und damit die Expression gestartet. Diese Temperatur liegt deutlich tiefer als für eine optimale Induktion des Lambda-P_{L}-Promotors erforderlich ist (42 °C). Uberraschenderweise zeigt sich, daß hierdurch die Enzymaktivitäten bzw. Proteinausbeuten nicht geschmälert werden, sondern bei Einhaltung aller erfindungsgemäßen Verfahrensparameter vielmehr deutlich gesteigert werden können.

Unmittelbar vor oder nach, vorzugsweise vor erfolgter Temperaturerhöhung werden nach dem erfindungsgemäßen verfahren aminosäurehaltige Substrate dem Kulturmedium zugesetzt. Als Substrate können dabei Mischungen aus einzelnen Aminosäuren, Hefeextrakte oder auch Gemische aus Aminosäuren und Hefeextrakt dienen.

Geeignet sind so beispielsweise Asparaginsäure, Glutaminsäure, Serin, Glutamin, Glycin, Threonin, Arginin, Alanin, Tyrosin, Tryptophan, Valin, Phenylalanin, Lysin, Leucin, Isoleucin und Prolin, aber auch andere natürlich vorkommende Aminosäuren sind einsetzbar. Dabei sind solche Gemische bevorzugt, die mindestens Phenylalanin, Tyrosin und Tryptophan enthalten. Zur Herstellung der Hefeextrakte (Suspension von Hefe in Wasser oder Puffer) können beliebige bekannte una im Handel erhältliche Hefekulturen eingesetzt werden.

Die Konzentration der zugesetzten Aminosäuren variiert erfindungsgemäß zwischen 0,05 g/l und 1,0 g/l je Aminosäure, vorzugsweise zwischen 0,1 g/l und 0,5 g/l. Der alternative Hefeextrakt wird in einer Konzentration von 1 g/l bis 10 g/l, vorzugsweise von 4 bis 6 g/l, zugesetzt. Besonders hohe Enzymaktivitäten/Proteinausbeuten können erzielt werden, wenn ein vollständiges Spektrum an Aminosäuren zugesetzt wird. Ersatzweise können auch Gemische aus Hefeextrakt und Aminosäuren, insbesondere den drei aromatischen Aminosäuren Phenylalanin, Tyrosin und Tryptophan eingesetzt werden.

Jedes einzelne erfindungsgemäße Verfahrensmerkmal bewirkt bereits einen mehr oder weniger ausgeprägten Effekt bezüglich einer gesteigerten Expressionsrate. Dies ist den unten aufgeführten Beispielen sowie den Abbildungen zu entnehmen. Die wirklich signifikanten Steigerungen der Expression auf das 5- bis 10fache des Vergleichswertes (Verfahrensparameter nach dem Stand der Technik) über einen längeren Zeitraum sind jedoch erst bei einer kombinierten Anwendung der drei erfindungsgemäßen Parameter - mittlere Biomasse, reduzierte Induktionstemperatur und Supplementierung von aminosäurehaltigen Substraten - zu beobachten. Nach dem erfindungsgemäßen Verfahren kann eine nennenswerte Expression über einen Zeitraum von 4 bis 8 Stunden aufrechterhalten werden, während im Vergleichsversuch die Expression bereits nach 2 bis 3 Stunden nahezu zum Erliegen kommt.

Nachdem die Expression weitgehend abgeschlossen ist, werden die Zellen nach Standardmethoden, wie z. B. in der EP-OS-02 90 768 oder in der EP-OS-03 07 730 beschrieben, aufgearbeitet, das exprimierte Protein in üblicher Weise isoliert und aufgereinigt und die Expressionsrate bzw. Enzymaktivität in bekannter Weise bestimmt. Dabei ist die angewandte Methodik jeweils von der Art des exprimierten Proteins abhängig.

Im folgenden sind die Abbildungen erläutert, in denen einzelne Teilbereiche der Erfindung dargestellt sind.

Die Abbildungen zeigen Diagramme, in denen die optische Dichte in OD-Einheiten bei 578 nm (a), die Enzymaktivität in Units/ml (b) und die biomassenspezifische Expressionsrate in Kilounits/OD/h (c) in Abhängigkeit zur Zeit in Stunden nach Beimpfung (Abszisse) bei wechselnden Verfahrensparametern dargestellt ist. In allen Fällen wird der gleiche rekombinante durch Temperaturinduktion zur Expression von Glucosedehydrogenase (Gluc-DH) befähigte E. coli-Wirtsstamm (siehe Text und Beispiel 1) kultiviert und aufgearbeitet.
- Fig. 1:: Expression von Gluc-DH gemäß den Versuchsbedingungen aus Beispiel 1 (Vergleichswert, Induktionstemperatur 42 °C, OD₅₇₈ = 2,5, ohne Zusätze).
- Fig. 2:: Wie 1, jedoch bei hoher Biomasse (OD₅₇₈ = 10).
- Fig. 3:: Wie 2, Induktionstemperatur: 39 °C.
- Fig. 4:: Expression von Gluc-DH bei mittlerer Biomasse (OD₅₇₈ = 2,5), Induktionstemperatur: 39 °C, Zusatz von Phenylalanin, Tyrosin und Tryptophan.
- Fig. 5:: Wie 4, jedoch Zusatz von Hefeextrakt ohne weitere Aminosäuren bei OD₅₇₈ = 5,0.
- Fig. 6:: Wie 5, jedoch zusätzlich Supplementierung von Phenylalanin, Tyrosin und Tryptophan.
- Fig 7:: Wie 4, jedoch vollständiges Spektrum von Aminosäuren bei OD₅₇₈ = 3,5.

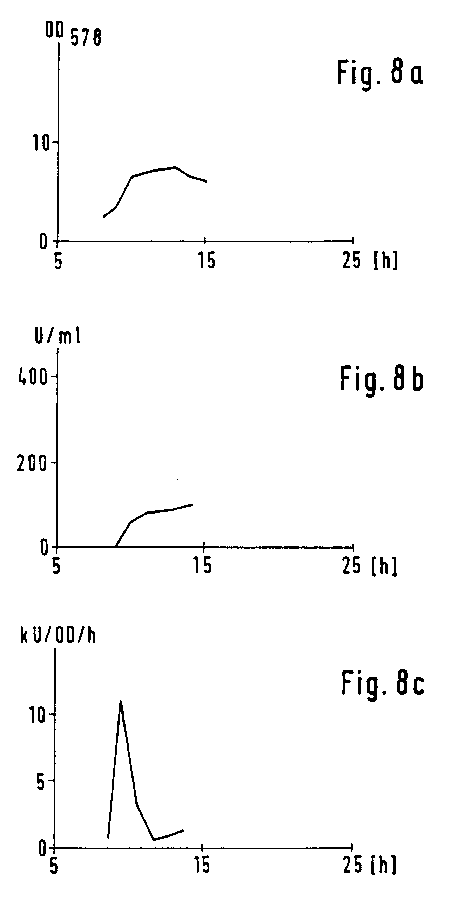

- Fig. 8:: Wie 6, jedoch Induktionstemperatur von 42 °C bei OD₅₇₈ = 2,5.

### Beispiel 1

E. coli N 100/pRK 248/pJH 115 (DSM 4047) wird in einer Nährlösung, enthaltend im wesentlichen

| | % (w/v) |
|---|---|
| Na₂HPO₄ x 2 H₂O | 0,19 |
| KH₂PO₄ | 0,075 |
| NH₄Cl | 0,4 |
| MgSO₄ x 7 H₂O | 0,1 |
| Citronensäure | 0,2 |
| Glucose | 0,1 |
| CaCl₂ x 2 H₂O | 0,01 |

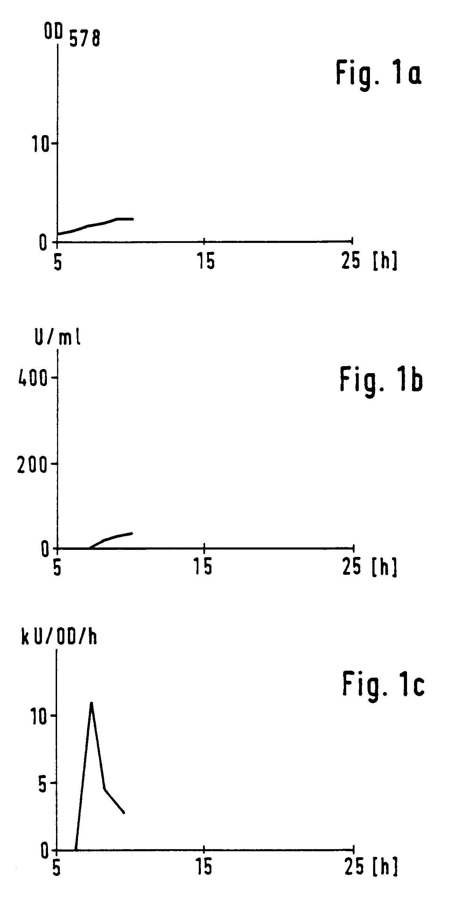

bei 32 °C in einem 1000-Liter-Fermenter kultiviert. Während der Wachstumsphase wird das Zellenwachstum durch regelmäßige Messung der optischen Dichte bei 578 nm verfolgt. Bei einer optischen Dichte von 2,5 (erreicht nach 7 bis 10 Stunden nach Beimpfung) wird die Temperatur auf 42 °C erhöht, und man läßt die Zellen weitere 5 h wachsen. In regelmäßigen Abständen wird die Glucosedehydrogenase-Aktivität nach dem z. B. in der EP-OS-02 90 768 angegebenen Aktivitätstest gemessen. Anschließend werden die Zellen bei 20 °C abzentrifugiert (5000 Upm), in einen Natriumphosphat-Puffer (0,1 Mol/l, pH 6,5) aufgenommen und unter Kühlung in einem Hochdruckhomogenisator bei 1000 bar homogenisiert. In das Homogenat werden 35 % PEG 1500 (Polyethylenglykol) innerhalb von 20 bis 25 Minuten eingerührt. Nach Auflösen des PEG werden 67 % (w/v Homogenat) des folgenden Phosphatpuffers unter Rühren bei pH 5,2 (± 0,1) zudosiert (Angaben in % w/w: NaH₂PO₄ x 2 H₂O 26,2 %, K₂HPO₄ 1,3 %, pH 4,7). Danach werden 6 % (w/v Homogenat) NaCl zugefügt und noch 2 h lang gerührt. Phasentrennung erfolgt durch Zentrifugation. Die Gluc-DH enthaltende obere Phase kann ggf. filtriert bzw. diafiltriert werden. Man erhält Glucosedehydrogenase mit einer Aktivität von 40 U/ml (Fig. 1a-c).

### Beispiel 2

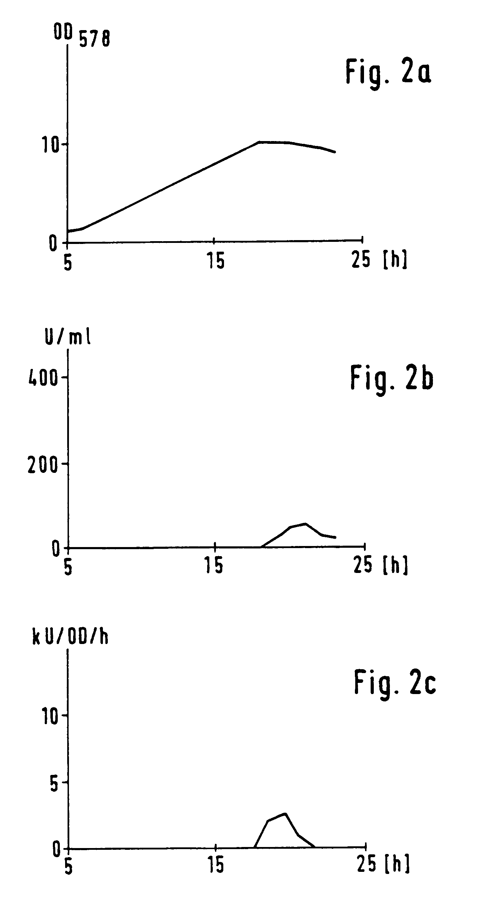

Aufzucht der rekombinanten Wirtszellen sowie Expression und Isolierung von Gluc-DH erfolgen analog Beispiel 1. Die Zellen werden jedoch wachsen gelassen bis eine optische Dichte von 10 erreicht ist (maximale Biomasse), ehe die Expression gestartet wird. Man erhält mit 50 U/ml eine geringfügig gesteigerte Enzymaktivität bzw. biomassenspezifische Expressionsrate (Fig. 2a-c).

### Beispiel 3

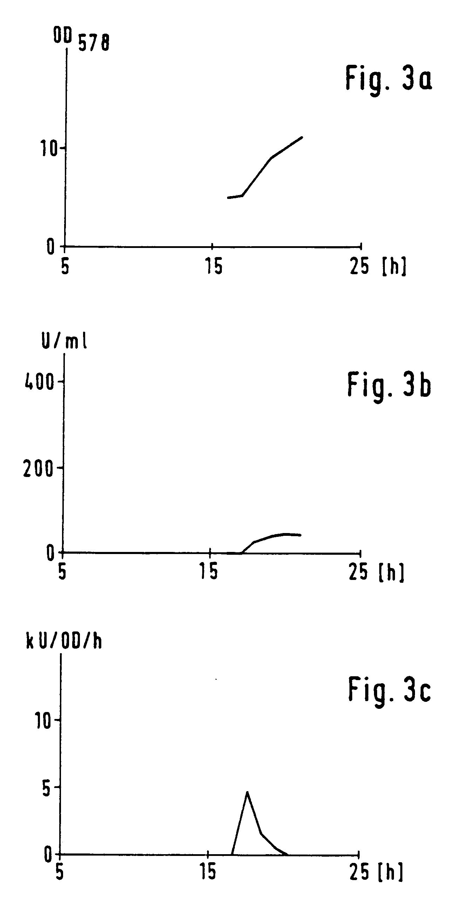

Aufzucht der rekombinanten Wirtszellen sowie Expression und Isolierung von Gluc-DH erfolgen analog Beispiel 2, jedoch wird eine Temperaturerhöhung lediglich auf 39 °C vorgenommen. Aktivitäten bzw. Ausbeuten entsprechen denen von Beispiel 2 (Fig. 3a-c).

### Beispiel 4

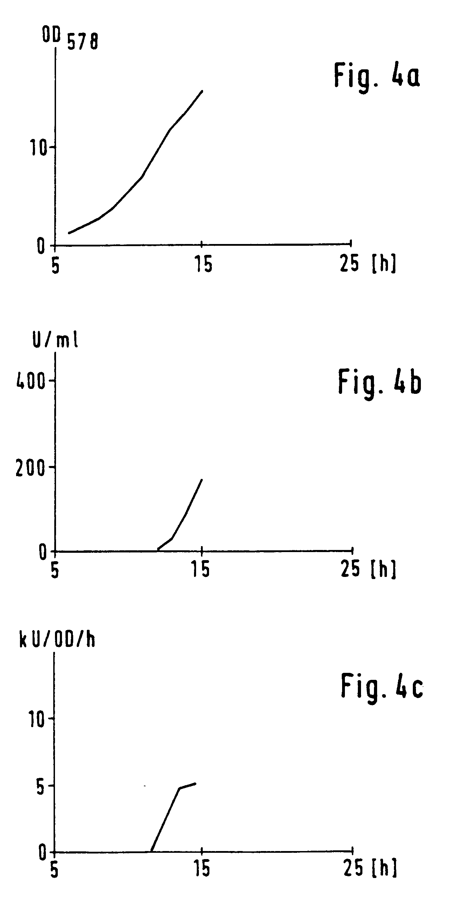

Aufzucht der rekombinanten Wirtszellen sowie Expression und Isolierung von Gluc-DH erfolgen analog Beispiel 1. Bei einer optischen Dichte OD = 2,5 erfolgt Zugabe von je 0,3 g/l Tryptophan, Tyrosin und Phenylalanin. Unmittelbar danach wird die Temperatur der Kulturbrühe auf 39 °C erhöht. Es wird eine Enzymaktivität von 170 U/ml erzielt (Fig. 4a-c).

### Beispiel 5

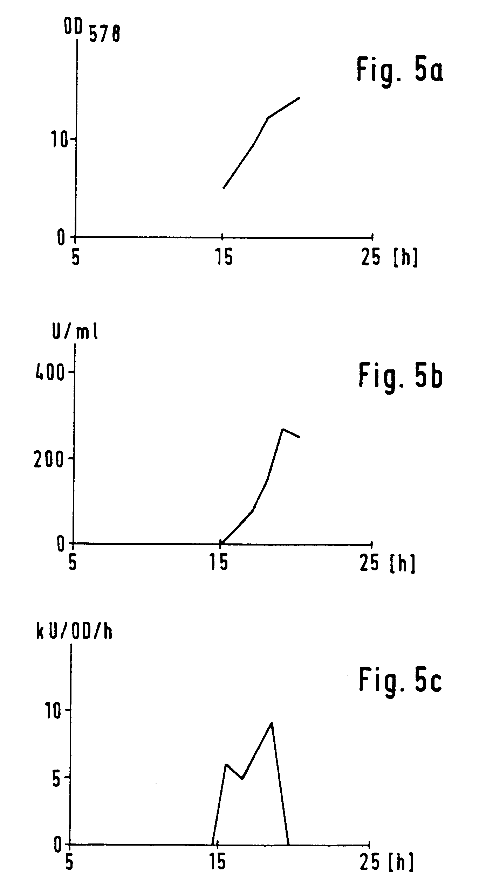

Aufzucht der rekombinanten Wirtszellen sowie Expression und Isolierung von Gluc-DH erfolgen analog Beispiel 4. Anstelle des Aminosäuregemisches wird ein Hefeextrakt (5 g/l) bei OD = 5,0 hinzugefügt. Es wird eine Enzymaktivität von 270 U/ml und eine maximale biomassenspezifische Expressionsrate von 9 kU/OD/h erzielt (Fig. 5a-c).

### Beispiel 6

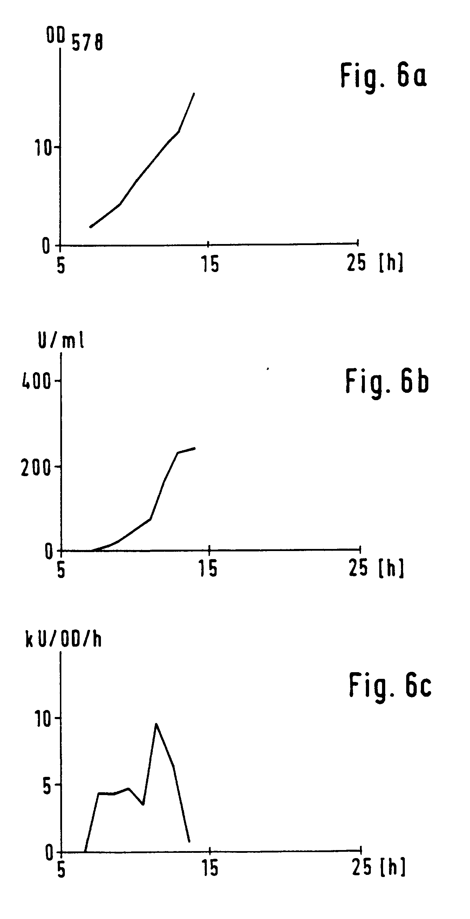

Aufzucht der rekombinanten Wirtszellen sowie Expression und Isolierung von Gluc-DH erfolgen analog Beispiel 4. Bei erreichter optischer Dichte OD = 2,0 wird vor Temperaturerhöhung auf 39 °C ein Hefeextrakt (5 g/l) und zusätzlich die Aminosäuren Phenylalanin, Tyrosin und Tryptophan in einer Konzentration von je 0,2 g/l hinzugegeben. Die Biosynthese von Gluc-DH hält über einen Zeitraum von 7 h an. Es läßt sich eine Aktivität von 240 U/ml erzielen (Fig. 6a-c).

### Beispiel 7

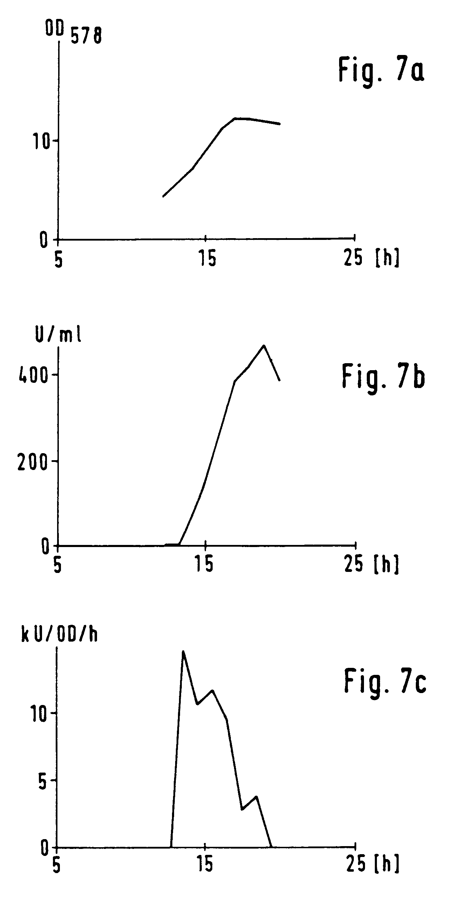

Aufzucht der rekombinanten Zellen sowie Expression und Isolierung von Gluc-DH erfolgen analog Beispiel 4. Bei erreichter optischer Dichte OD = 3,5 wird vor Temperaturerhöhung auf 39 °C ein Gemisch aller natürlich vorkommenden Aminosäuren in einer Konzentration von je 0,1 g/l hinzugegeben. Man erzielt eine Aktivität von 460 U/ml und eine maximale biomassenspezifische Expressionsrate von etwa 14 kU/OD/h. Auch hier bleibt die Biosyntheseaktivität etwa 7 h bestehen (Fig. 7a-c).

### Beispiel 8

Aufzucht der rekombinanten Zellen sowie Expression und Isolierung von Gluc-DH erfolgen analog Beispiel 6. Nach Zugabe der entsprechenden Substrate bei OD = 2,5 wird die Temperatur jedoch auf 42 °C erhöht. Man beobachtet, wie in Beispiel 1, eine kurzzeitig hohe, dann aber stark nachlassende Biosyntheserate, die zu einer Enzymaktivität von 95 U/ml führt.

## Patentansprüche

1. Verfahren zur Herstellung von Proteinen durch Kultivierung rekombinanter Wirtszellen in einer Nährlösung und temperaturinduzierte Expression durch den lambda-P_{L}-Promotor und nachfolgende Isolierung der Proteine, dadurch gekennzeichnet, daß die Expression der Proteine
(a) bei einer Biomasse der rekombinanten Wirtszellen, die 5 bis 40 % der maximal erreichbaren Biomasse dieser Wirtszellen entspricht,
(b) bei einer Induktions- bzw. Expressionstemperatur von unterhalb 40°C, und
(c) in Anwesenheit von unmittelbar vor oder nach der Induktion zugesetzten Aminosäuren und/oder Hefeextrakten,
durchgeführt wird.

2. Verfahren nach Anspruch 1, dadurch gekennzeichnet, daß die Biomasse der verwendeten Wirtszellen 10 - 20% der maximal erreichbaren Biomasse entspricht.

3. Verfahren nach Anspruch 1 oder 2, dadurch gekennzeichnet, daß die Induktions- bzw. Expressionstemperatur 38 bis 39°C beträgt.

4. Verfahren nach einem der Ansprüche 1 bis 3, dadurch gekennzeichnet, daß das zugesetzte Gemisch aus Aminosäuren mindestens Phenylalanin, Tyrosin und Tryptophan enthält.

5. Verfahren nach Anpruch 4, dadurch gekennzeichnet, daß das Gemisch zusätzlich einen Hefeextrakt enthält.

6. Verfahren nach einem der Ansprüche 1 bis 5, dadurch gekennzeicnet, daß als rekombinante Wirtszellen *E. coli*-Zellen verwendet werden.

7. Verfahren nach einem der Ansprüche 1 bis 6 zur Herstellung von rekombinanter Glucosedehydrogenase.

## Claims

1. Process for the preparation of proteins by cultivating recombinant host cells in a nutrient solution and by thermally-induced expression through the lambda P_{L} promoter and subsequent isolation of the proteins, characterized in that the expression of the proteins is carried out
a) at a biomass of the recombinant host cells which is equivalent to 5 to 40 % of the maximum achievable biomass of these host cells,
b) at an induction or expression temperature below 40°C, and
c) in the presence of amino acids and/or yeast extracts added immediately before or after the induction.

2. Process according to Claim 1, characterized in that the biomass of the host cells used is equivalent to 10 - 20 % of the maximum achievable biomass.

3. Process according to Claim 1 or 2, characterized in that the induction or expression temperature is 38 to 39°C.

4. Process according to any of Claims 1 to 3, characterized in that the added mixture of amino acids contains at least phenylalanine, tyrosine and tryptophan.

5. Process according to Claim 4, characterized in that the mixture additionally contains a yeast extract.

6. Process according to any of Claims 1 to 5, characterized in that the recombinant host cells used are *E. coil* cells.

7. Process according to any of Claims 1 to 6 for the preparation of recombinant glucose dehydrogenase.

## Revendications

1. Procédé pour préparer des protéines par culture de cellules-hôtes recombinantes dans une solution nutritive, expression induite en fonction de la température par le promoteur lambda-P_{L} et isolement subséquent des protéines, caractérisé en ce que l'expression des protéines est réalisée
(a) pour une biomasse des cellules-hôtes recombinantes représentant 5 à 40 % de la biomasse maximale possible desdites cellules-hôtes,
(b) à une température d'induction ou d'expression inférieure à 40 °C, et
(c) en présence d'aminoacides et/ou d'extraits de levure ajouté directement avant ou après l'induction.

2. Procédé selon la revendication 1, caractérisé en ce que la biomasse des cellules-hôtes utilisées représente 10 à 20 % de la biomasse maximale possible.

3. Procédé selon la revendication 1 ou 2, caractérisé en ce que la température d'induction ou d'expression est de 38 à 39 °C.

4. Procédé selon l'une quelconque des revendications 1 à 3, caractérisé en ce que le mélange d'aminoacides ajoutés contient au moins de la phénylalanine, de la tyrosine et du tryptophane.

5. Procédé selon la revendication 4, caractérisé en ce que le mélange contient en plus un extrait de levure.

6. Procédé selon l'une quelconque des revendications 1 à 5, caractérisé en ce que les cellules-hôtes recombinantes utilisées sont des cellules d'E. coli.

7. Procédé selon l'une quelconque des revendications 1 à 6 pour préparer de la glucosedéshydrogénase recombinante.
